# EUROPÄISCHE PATENTANMELDUNG

(11) **EP 3 925 629 A1**
(43) Veröffentlichungstag der Anmeldung: **22.12.2021**
(21) Anmeldenummer: 20180595.9
(22) Anmeldetag: 17.06.2020
(51) Int. Cl.: A61L 2/10, A61L 2/24, B62B 3/00

(54) **VORRICHTUNG ZUR DESINFEKTION**

(71) Anmelder: Planlicht GmbH & Co. KG, 6134 Vomp (AT)
(72) Erfinder: Mayr, Raimund, 6134 Vomp (AT)
(74) Vertreter: Schwarz & Partner Patentanwälte GmbH

(57) **Zusammenfassung**

Die Erfindung betrifft ein Verfahren und eine Vorrichtung zur Desinfektion von Gegenständen, vorzugsweise Wagen wie Einkaufswagen, Schubwagen, Transportwagen oder Gepäckwagen mittels UV-Licht, wobei zumindest drei Desinfektionsstationen (d) vorgesehen sind, wobei jede der Desinfektionsstationen (d) - vorzugsweise mehrere - UV-Lichtmittel (7), einen Desinfektionsinnenraum (6) und eine verschließbare Türe (5), vorzugsweise ein Rolltor an der Vorderseite umfasst, wobei die UV-Lichtmittel (7) an den Innenwänden der Desinfektionsstation (d) angebracht sind, wobei die zumindest drei Desinfektionsstationen (d) jeweils in eine Aufnahmestation (1) zur Aufnahme benutzter Gegenstände, vorzugsweise Wagen, eine Entnahmestation (3) zur Entnahme entkeimter Gegenstände, vorzugsweise Wagen und eine Entkeimungsstation (2) zur Desinfektion der Gegenstände, vorzugsweise Wagen aufgeteilt werden, wobei während die Tür (5) der Entkeimungsstation (2) geschlossen ist, die Türen (5) der Entnahmestation (3) und der Aufnahmestation (1) geöffnet sind, wobei bei geöffneter Türe (5) die Lichtabgabe durch die UV-Lichtmittel (7) deaktiviert und bei geschlossener Türe (5) die Lichtabgabe durch die UV-Lichtmittel (7) zumindest zeitweise aktiviert wird.

## Beschreibung

Die Erfindung betrifft ein Verfahren und eine Vorrichtung zur Desinfektion von Wagen wie Einkaufswagen, Schubwagen, Transportwagen oder Gepäckwagen mittels UV-Licht mit zumindest drei Desinfektionsstationen.

### HINTERGRUND DER ERFINDUNG

Im Zuge der COVID-19-Pandemie ist die Desinfektion von Alltagsgegenständen, welche von einer Vielzahl von Personen berührt und benützt werden, in den Fokus gerückt. Es gibt einige Untersuchungen im Zusammenhang mit Einkaufswagen, die belegen, dass diese mit einer Vielzahl von Krankheitserregern wie Viren und Bakterien kontaminiert sein können. Daher müssen Einkaufswagen täglich mehrmals und vor allem auch kurzfristig desinfiziert werden.

Eine Möglichkeit der Desinfektion ist durch Desinfektionsmittel gegeben, mit welchen die Handgriffe der Einkaufswagen vor bzw. nach jedem Gebrauch desinfiziert werden. Eine kontrollierte Desinfektion ist damit jedoch sehr schwer umsetzbar, da im Normalfall die Einkaufswagen von den Kunden selbst vor dem Gebrauch mehr oder weniger gründlich desinfiziert werden. Außerdem stellt eine solche Art der Desinfektion Geschäfte vor eine logistische Herausforderung, da zu jedem Zeitpunkt genügend Desinfektionsmittel und Reinigungstücher vorhanden sein müssen.

Weiters erlaubt der Einsatz von Desinfektionsmittel nie eine Desinfektion des gesamten Einkaufswagens. Im Normalfall wird immer nur der Handgriff desinfiziert, wobei eine fehlerhafte Desinfektion sehr wahrscheinlich ist, da jeder Kunde die Desinfektion unterschiedlich durchführt. Daher bietet eine Desinfektion durch Desinfektionsmittel keine praktische, umfassende und komplikationsfreie Möglichkeit Krankheitserreger an Einkaufswagen abzutöten.

Eine wesentlich bessere Methode der Desinfektion von Einkaufswagen, Transportwagen, Gepäckwagen und ähnlichen Gegenständen bietet die Bestrahlung mittels UV-C Licht. Durch geschickte Bestrahlung können auch schwer erreichbare Bereiche zuverlässig entkeimt werden.

Desinfektionsvorrichtungen, welche eine Bestrahlung von Einkaufswagen mit UV-Licht ermöglichen, werden beispielsweise in KR 10-0936552 B1, DE 10 2009 040 765 A1 und US 2006/0186358 A1 offenbart. Die in diesen Dokumenten beschriebene Bestrahlung der Einkaufswagen mit UV-Licht ist sehr zeitaufwändig und eine vollständige Desinfektion der Einkaufswagen nicht möglich. In US 2008/0178412 A1 wird außerdem eine sehr aufwändige Waschanlage zur Desinfektion von Einkaufswagen offenbart, welche eine Entkeimung der Einkaufswagen in mehreren Schritten ermöglicht. Eine Bestrahlung mittels UV-Licht kann als letzter Schritt in der Waschanlage vorgesehen sein. Die aufwendige Anlage erlaubt jedoch keine kurzfristige und schnelle Desinfektion der Einkaufswagen.

Bei den im Stand der Technik bekannten UV-Desinfektionsvorrichtungen für Einkaufswagen braucht es im Normalfall eine Person, welche die Einkaufswagen in die Desinfektionsvorrichtung schiebt oder stellt und die Desinfektion startet. Das heißt Geschäfte bzw. Infrastrukturen, in welchen Transport- oder Einkaufswagen verwendet werden, müssten zusätzliches Personal zur Desinfektion benutzter Einkaufswagen zur Verfügung stellen. Außerdem unterscheiden sich die bekannten Desinfektionsvorrichtungen von den üblichen Parkboxen bzw. Reihenanfangsstationen, aus welchen Einkaufswagen normalerweise entnommen bzw. abgestellt werden. Die im Stand der Technik beschriebenen Vorrichtungen würden daher bei den Kunden einerseits zu Komplikationen in der Handhabung mit resultierenden fehlerhaften Desinfektionen und andererseits zu längeren Wartezeiten führen, da die Einkaufswagen vor der Benutzung erst in die UV-Desinfektionsvorrichtung gestellt werden müssten bzw. nicht genügend bereits desinfizierte Einkaufswagen vorhanden wären.

### KURZBESCHREIBUNG DER ERFINDUNG

Aufgabe der vorliegenden Erfindung ist daher ein Verfahren zur Desinfektion von Gegenständen, insbesondere Wagen wie Einkaufswagen, Schubwagen, Transportwagen oder Gepäckwagen bereitzustellen, welches in das bekannte System zur Entnahme und Rückgabe der Wagen einfach integrierbar ist und eine schnelle und effiziente Entkeimung benutzter Wagen in einem automatisch gesteuerten Ablauf erlaubt.

Gelöst wird diese Aufgabe durch ein Verfahren zur Desinfektion von Gegenständen, insbesondere Wagen wie Einkaufswagen, Schubwagen, Transportwagen oder Gepäckwagen mittels UV-Licht,
wobei zumindest drei Desinfektionsstationen vorgesehen sind,
wobei jede der Desinfektionsstationen - vorzugsweise mehrere - UV-Lichtmittel, einen Desinfektionsinnenraum und eine verschließbare Türe, vorzugsweise ein Rolltor an der Vorderseite, umfasst,
wobei die UV-Lichtmittel an den Innenwänden der Desinfektionsstation angebracht sind, dadurch gekennzeichnet, dass
die zumindest drei Desinfektionsstationen jeweils in eine Aufnahmestation zur Aufnahme benutzter Gegenstände, vorzugsweise Wagen, eine Entnahmestation zur Entnahme entkeimter Gegenstände, vorzugsweise Wagen und eine Entkeimungsstation zur Desinfektion der Gegenstände, vorzugsweise Wagen aufgeteilt werden,
wobei während die Tür der Entkeimungsstation geschlossen ist, die Türen der Entnahmestation und der Aufnahmestation geöffnet sind,
wobei bei geöffneter Türe die Lichtabgabe durch die UV-Lichtmittel deaktiviert und bei geschlossener Türe die Lichtabgabe durch die UV-Lichtmittel zumindest zeitweise aktiviert wird, wobei weiters die Schritte vorgesehen sind:
   - Einschieben benutzter Gegenstände, vorzugsweise Wagen in die Aufnahmestation, wobei die Gegenstände, vorzugsweise Wagen vorzugsweise ineinandergeschoben werden und Entnahme von entkeimten Wagen aus der Entnahmestation, während in der Entkeimungsstation benutzte Gegenstände, vorzugsweise Wagen mit von den UV-Lichtmitteln abgestrahltem UV-Licht bestrahlt werden, solange bis entweder die maximale Ladekapazität der Aufnahmestation erreicht ist oder die Entnahmestation keine Wagen mehr beinhaltet,
   - Schließen der Türe der Aufnahmestation und Aktivierung der UV-Lichtmittel und Öffnen der Türe der Entkeimungsstation,
   - Starten bei Schritt eins, wobei die vormalige Entnahmestation der Aufnahmestation, die vormalige Aufnahmestation der Entkeimungsstation und die vormalige Entkeimungsstation der Entnahmestation entspricht.

Die zumindest drei Desinfektionsstationen entsprechen dabei im Wesentlichen einer Parkbox oder Reihenanfangsstation für Einkaufs-, Schub-, Transport- oder Gepäckwagen, welche zusätzlich die UV-Lichtmittel, eine Leuchte zur normgerechten Ausleuchtung des Innenraumes, eine Steuerungseinheit und eine verschließbare Türe umfassen. Die Desinfektionsstationen umfassen dabei einen Desinfektionsinnenraum, welcher durch die Wände der Desinfektionsstation begrenzt ist. Das erfindungsgemäße Verfahren bzw. die Verfahrensschritte ermöglichen dem Nutzer ein gewohntes Handling der Wagen, bei welchen es sich beispielweise um Einkaufswagen handelt. Wie bei den üblichen Parksystemen für Einkaufswagen entnimmt der Nutzer einen Einkaufswagen aus einer Reihe und stellt einen benutzten Einkaufswagen in Längsrichtung wiederum in einer Reihe ab, wobei der Einkaufswagen soweit vorgeschoben wird, bis er mit dem sich davor befindlichen Einkaufswagen ineinandergeschoben ist. Der einzige Unterschied des erfindungsgemäßen Verfahrens zu diesem gewohnten Handling ist, dass immer nur eine bestimmte (wechselnde) Reihe zur Entnahme der Einkaufswagen und eine bestimmte (wechselnde) Reihe zu Rückgabe der Einkaufswagen vorgesehen ist.

Ein wesentlicher Vorteil des erfindungsgemäßen Verfahrens zur Desinfektion von Wagen wie Einkaufswagen, Schubwagen, Transportwagen oder Gepäckwagen mittels UV-Licht ist, dass sich mit dem Verfahren für Nutzer keinerlei Wartezeiten ergeben. Aufgrund der Verwendung von zumindest drei Desinfektionsstationen, kann garantiert werden, dass zu jeder Zeit desinfizierte Wagen vorhanden sind. Ein Nutzer muss daher weder den Wagen selbstständig desinfizieren noch darauf warten, dass der Wagen desinfiziert wird. Daraus ergibt sich auch für das Unternehmen kein Personalaufwand, da die Steuerungseinheit das Verfahren vollkommen selbsttätig und sicher steuert.

In einer besonders bevorzugten Ausführungsvariante umfasst das Verfahren die Betätigung eines Signalmittels durch eine Steuerungseinheit, welches den Status der Desinfektionsstationen ausgibt. Bevorzugt ist das Signalmittel ein visuelles Signalmittel. Das Signalmittel kann an der Vorderseite jeder der zumindest drei Desinfektionsstationen angebracht sein und zumindest zwei verschiedene visuelle Signale abgeben. Die visuellen Signalmittel können Anzeigetafeln umfassen, welche anzeigen, ob eine Desinfektionsstation gerade die Funktion der Aufnahme-, Entnahme- oder Entkeimungsstation übernimmt. Die Steuerungseinheit schaltet dabei zeitgleich zur Schließung bzw. Öffnung der Türe der Aufnahme- bzw. Entkeimungsstation das visuelle Signalmittel bzw. die Anzeigetafel um, sodass wiederum klar erkenntlich ist, in welche der zumindest drei Desinfektionsstationen benutze Einkaufswagen abgestellt und aus welcher der zumindest drei Desinfektionsstationen entkeimte Einkaufswagen entnommen werden können. Beispielsweise können die Aufnahme- und Entnahmestationen durch Symbole wie Pfeile auf den Anzeigetafeln gekennzeichnet werden. Ein in die Desinfektionsstation zeigender Pfeil könnte dabei die Aufnahmestation kennzeichnen und ein aus der Desinfektionsstation zeigender Pfeil die Entnahmestation. Zusätzlich zur geschlossenen Türe kann auch die Entkeimungsstation noch mit einem visuellen Signal wie z.B. einem Stoppzeichen oder mit einem Ampelsignal gekennzeichnet werden.

Die - vorzugsweise visuellen - Signalmittel ermöglichen eine sehr einfache Realisierung eines automatisierten Verfahrens zur Desinfektion von Einkaufs-, Schub-, Transport- oder Gepäckwagen mittels UV-Licht, für welches kein Personal zur Durchführung der Desinfektion notwendig ist. In einer speziellen Ausführungsvariante besteht die Möglichkeit die visuellen Signalmittel durch Audiosignale zu ergänzen.

Der Ablauf wird dabei nur durch die Steuerungseinheit geregelt. Die Ablaufsteuerung umfasst dabei das Öffnen und Schließen der Tore und das dazugehörige Steuern der Signalmittel sowie die Steuerung der UV-Lichtmittel, der Allgemeinbeleuchtung, der Sensor-Signale und sicherheitstechnischer Funktionen.

Bevorzugt werden für die Steuerung des Ablaufes in jeder der Desinfektionsstationen Lichtschranken vorgesehen, die jeweils ein Signal ausgeben, wenn eine Desinfektionsstation mit Einkaufs-, Schub-, Transport- oder Gepäckwagen voll ist, bzw. wenn alle Wagen entnommen sind. Darüber hinaus kann jede Desinfektionsstation mit einem Bewegungsmelder versehen sein, um eine Ansteuerung der Tür zu verhindern, solange sich Personen im Desinfektionsinnenraum aufhalten. Die Signale aller Sensoren werden in der übergeordneten Ablaufsteuerung ausgewertet. Die Ansteuerung der Türen und der Signalmittel erfolgt bevorzugt gleichzeitig, wenn die Entnahmestation leer ist, oder die Aufnahmestation voll ist und sobald kein Signal des Bewegungsmelders im Innenraum einer der Stationen erfasst wird.

Die Steuerung für die einzelnen Desinfektionsstationen kann durch die mit der Türsteuerung verbundene Sicherheitsschaltung getriggert werden. Bei geöffneter Türe kann nur die Allgemeinbeleuchtung aktiviert werden, während die UV-Bestrahlung durch Schließen der Türe gestartet werden kann. Besonders bevorzugt erfolgt die UV-Bestrahlung zeitgesteuert, wobei die Zeitdauer einstellbar ist. Die Intensität der UV-Bestrahlung kann über einen integrierten UV-Sensor aktiv auf ein konstantes Niveau geregelt werden. Somit sind unterschiedliche Desinfektionsprogramme realisierbar, und es ist sichergestellt, dass die gewünschte UV-Dosis erreicht wird. In einer anderen Ausführungsvariante kann die Intensitätssteuerung über eine Constant-Light-Output-Funktion der LED-Treiber erfolgen, um den lebensdauerbedingten Strahlungsrückgang der LEDs auszugleichen und eine annähernd konstante UV-Dosis sicherzustellen.

Ein weiterer Vorteil des erfindungsgemäßen Verfahrens mit zumindest einer Aufnahme-, Entnahme- und Entkeimungsstation ist die Möglichkeit einer langen UV-Bestrahlungszeit. Die Wagen in der Entkeimungsstation können im Wesentlichen solange bestrahlt werden, bis die maximale Ladekapazität der Aufnahmestation erreicht ist bzw. der letzte Einkaufswagen aus der Entnahmestation entnommen wurde. Da die Desinfektionsstationen je nach Länge bis zu 40 Einkaufswagen aufnehmen können, kann diese Zeitspanne abhängig vom Nutzeraufkommen relativ lang sein. Alternativ kann die Dauer der Bestrahlung auch über die Steuerungseinheit eingestellt und nach einer gewissen Zeit beendet werden.

Die Erfindung betrifft außerdem eine Vorrichtung zur Desinfektion von Gegenständen, insbesondere Einkaufswagen, Schubwagen, Transportwagen oder Gepäckwagen mittels UV-Licht, umfassend zumindest drei Desinfektionsstationen, wobei jede der Desinfektionsstationen
- einen Desinfektionsinnenraum,
- eine verschließbare Türe, vorzugsweise ein Rolltor an der Vorderseite,
- mehrere UV-Lichtmittel, welche an den Innenwänden angebracht sind,
- und eine Steuerungseinheit
   umfasst,
   wobei die Steuerungseinheit im Betriebszustand
   (i) die drei Desinfektionsstationen in Aufnahmestation, Entnahmestation und Entkeimungsstation unterteilt;
   (ii) die Tür der Entkeimungsstation geschlossen hält und die Türen der Aufnahmestation und der Entnahmestation geöffnet hält;
   (iii) die Lichtabgabe der UV-Lichtmittel steuert, wobei bei geöffneter Türe die Lichtabgabe durch die UV-Lichtmittel deaktiviert ist und wobei bei geschlossener Türe die Lichtabgabe durch die UV-Lichtmittel zumindest zeitweise aktiviert ist.

Bevorzugt sind die Desinfektionsstationen derart ausgeführt, dass genau eine Reihe von Wagen, z.B. Einkaufswagen in einer Station Platz findet. Natürlich können die Desinfektionsstationen auch sehr einfach derart ausgeführt werden, dass zwei oder mehr Reihen von Einkaufswagen in einer Station Platz haben. Da jedoch zumindest drei Desinfektionsstationen nebeneinander angeordnet werden, würde somit die Gesamtgröße der Vorrichtung auch um einiges größer werden.
Die Breite der Desinfektionsstationen kann daher zwischen 0,5 und 1,5 m sein, während die Höhe zwischen 1,5 und 2,5 m sein kann. Die Höhe der Desinfektionsstationen ist dabei größer als die Höhe der Wagen, um ein komfortables Betreten der Stationen durch Personen zu ermöglichen. Speziell bei wenigen Wagen in einer Desinfektionsstation muss eine Person zur Entnahme bzw. Abgabe eines Wagens bis ans Ende der Desinfektionsstation gehen. Die Länge der Stationen ist variabel und hängt von der Anzahl der Wagen ab, welche von den zumindest drei Desinfektionsstationen aufnehmbar sein soll. Bevorzugt weisen die Desinfektionsstationen eine Länge von 4 bis 8 m auf.

Die zumindest drei Desinfektionsstationen werden bevorzugt in Längsrichtung nebeneinander angeordnet. Dadurch ergibt sich ein sehr geringer Platzbedarf für die gesamte Vorrichtung.

Bevorzugt umfasst jede der Desinfektionsstationen eine Allgemeinbeleuchtung zur Ausleuchtung des Innenraums. Die Allgemeinbeleuchtung kann in eines oder mehrere UV-Lichtmittel integriert werden oder auch extern an einer der Seitenwände oder der Deckenwand angebracht werden.

In einer weiteren Ausführungsvariante steuert die Steuerungseinheit auch das Schließen und Öffnen der Türe, wobei die Türe der Aufnahmestation schließt sobald entweder die maximale Ladekapazität der Desinfektionsstation erreicht ist oder die Entnahmestation leer ist, wobei die Tür der Entkeimungsstation öffnet sobald die Tür der Aufnahmestation schließt, wobei die Entkeimungsstation dann zur Entnahmestation, die Aufnahmestation zur Entkeimungsstation und die Entnahmestation zur Aufnahmestation wird.

Zusätzlich kann über die Steuerungseinheit geregelt werden, dass auch die Türen der Entnahme- und Aufnahmestation nur während der Öffnungszeiten eines Geschäfts öffnen. Beispielsweise können alle Türen in der Nacht bzw. bei Geschäftsschluss schließen und am Morgen bzw. bei Geschäftsöffnung wieder öffnen. Dies erlaubt einen Schutz der Wagen vor Diebstahl sowie auch einen erhöhten Schutz vor äußerer Verschmutzung wie z.B. Pollen an den Einkaufswagen.

In einer bevorzugten Ausführungsvariante besteht die Steuerungseinheit im Wesentlichen aus zwei Teilen: (1) einer übergeordneten Ablaufsteuerung der Türen und der Signalmittel der gesamten Anlage, und (2) einer für jede der drei Desinfektionsstationen autarken Steuerung für die UV-C Bestrahlung, für die Allgemeinbeleuchtung des Innenraumes, für die Bereitstellung von Sensor-Signalen für die übergeordnete Ablaufsteuerung und für sicherheitstechnische Funktionen. Die übergeordnete Ablaufsteuerung kann dabei in einer der Steuerungseinheiten der zumindest drei Desinfektionsstationen inkludiert sein.

Die Steuerungseinheit erlaubt somit eine geregelte Durchführung der Desinfektion benutzter Wagen, welche ohne Personal durchgeführt wird. Dabei können die Nutzer wie gewohnt Einkaufswagen abstellen und holen, ohne sonstige Schritte ausführen zu müssen. Die einzige Änderung zum gewohnten Ablauf ist, dass die Einkaufswagen in bestimmten Parkboxen abgestellt und aus bestimmten Parkboxen entnommen werden müssen, während andere Parkboxen für eine gewisse Zeit verschlossen sind. Es muss also weder das Personal noch die Kunden eines Supermarkts oder sonstigen Geschäfts sich um die Desinfektion der Einkaufswagen kümmern bzw. diese durchführen.

In einer Ausführungsvariante können die Wände der Desinfektionsstationen modulare Einzelsegmente umfassen, welche sehr einfach montierbar sind und den Desinfektionsinnenraum in seiner Größe beliebig skalierbar machen.

Besonders bevorzugt umfasst jede der zumindest drei Desinfektionsstation ein Signalmittel, welches zumindest zwei verschiedene Signale abgeben kann, an der Vorderseite, welches von der Steuerungseinheit gesteuert wird. Dieses Signalmittel gibt an, ob es sich um eine Entnahme-, Aufnahme- oder Entkeimungsstation handelt.

Bei dem Signalmittel kann es sich um ein visuelles Signalmittel handeln. Das visuelle Signalmittel kann beispielsweise eine Anzeigetafel umfassen, auf welcher unterschiedliche Symbole angezeigt werden. In einer Ausführungsvariante kann ein Pfeil, welcher in den Innenraum einer Desinfektionsstation weist, die Aufnahmestation, ein Pfeil, welcher in die Gegenrichtung weist, die Entnahmestation und ein Stoppsymbol die Entkeimungsstation anzeigen. Auf jeder der Anzeigetafeln können dabei alle verschiedenen Symbole angezeigt werden, da sich beim Schließen und Öffnen der Türen der zumindest drei Desinfektionskammern deren Funktion ändert.

In einer bevorzugten Ausführungsvariante ist an jeder der Innenwände des Desinfektionsinnenraums zumindest ein UV-Lichtmittel angebracht. Im Wesentlichen kann jegliche Kombination einer Anbringung der UV-Lichtmittel an den Innenseiten der den Innenraum einschließenden Wände vorgesehen werden und anwendungsspezifisch umgesetzt werden.

Je mehr Lichtmittel an den Wänden angebracht werden, desto schneller und umfassender ist die Desinfektion in der erfindungsgemäßen Desinfektionsstation. Genauer gesagt, reduziert sich die zur Entkeimung notwendige Zeit direkt proportional mit der Anzahl der verwendeten UV-Lichtmittel. Bevorzugt werden die UV-Lichtmittel derart angebracht, dass im Wesentlichen alle Bereiche der Wagen in der Entkeimungsstation von der UV-Strahlung erreicht werden. Somit kann eine schnelle und vollständige Desinfektion durchgeführt werden.

Bei dem von den UV-Lichtmitteln ausgesandten Licht kann es sich um UV-C Licht mit einer Wellenlänge von vorzugsweise 250 bis 280 nm handeln. In diesem Wellenlängenbereich ist die entkeimende Wirkung des UV-Lichtes am stärksten. Die Strahlung wird von DANN oder RNA der Mikroorganismen sehr stark absorbiert und dadurch geschädigt. Aufgrund der geschädigten DNA oder RNA sind die Mikroorganismen nicht mehr Replikationsfähig und nicht mehr infektiös.

In einer bevorzugten Ausführungsform umfassen die UV-Lichtmittel LED-Module, welche zumindest eine UV-LED, vorzugsweise mehrere UV-LEDs, aufweisen. Zusätzlich kann das LED-Modul zumindest eine LED zur Abgabe von Licht im sichtbaren Spektralbereich umfassen. Die einzelnen LED-Module können beispielsweise auf einem Aluminiumträgerprofil angebracht werden. Das Trägerprofil dient einerseits der Kühlung der LED-Module und lässt sich andererseits sehr einfach an den Wänden im Innenraum anbringen. Die hintereinander am Trägerprofil angebrachten LED-Module können seriell verschaltet werden. Durch die Verwendung einzelner Module lässt sich die Strahlungsquelle in Längsrichtung sehr einfach skalieren und an unterschiedliche Wandbreiten anpassen. Zur Optimierung der abgegebenen UV-Strahlung können auch mehrere Trägerprofile untereinander an einer Wand angebracht werden.

Bei Ausfall eines LED-Moduls lässt sich dieses auch auf einfache Weise tauschen. Bevorzugt sind die mehreren UV-LEDs auf einem LED-Modul parallel verschaltet und die LED zur Abgabe von sichtbarem Licht ist seriell dazu geschaltet. Bei der LED kann es sich um eine blaue LED handeln. Die vorzugsweise blaue LED dient der Betriebssicherheit, um eine eingeschaltete UV-Strahlungsquelle deutlich zu erkennen. Indem jedes LED-Module eine sichtbare LED umfasst, lässt sich die Abstrahlung jedes einzelnen LED-Moduls überprüfen.

Besonders bevorzugt umfassen die LED-Module UV-LEDs im UV-C Bereich von etwa 250 bis 280 nm. Die genaue Anzahl, Leistung und Positionierung der LED-Module kann je nach Anwendung sehr einfach skaliert und geändert werden. Die erfindungsgemäßen Desinfektionsstationen lassen sich daher anwendungsspezifisch gestalten. Durch die Verwendung der LED-Module ergibt sich außerdem eine sehr hohe Lichtpunktanzahl, welche Schattenbildungen auf ein Minimum reduziert. Daher werden alle Bereiche der zu entkeimenden Gegenstände in der Desinfektionskammer von der UV-Strahlung erreicht. Zusätzlich können die LED-Module auf den Trägerprofilen in einfacher Weise allseitig in den Desinfektionsinnenräumen montiert werden, um Schattenbildungen noch mehr zu verhindern.

In einer weiteren Ausführungsvariante umfasst die Steuerungseinheit eine Sicherheitsschaltung, welche die Lichtabgabe der UV-Lichtmittel bei offener Türe unterbindet. Bevorzugt kann mit der Steuerungseinheit die Abgabe der UV-Strahlung nur bei geschlossener Türe gestartet werden. Bei geöffneter Türe bzw. wenn die Türe geöffnet wird, kann die Steuerungseinheit aber die optionale Allgemeinbeleuchtung aktivieren. Außerdem unterbricht die Sicherheitsschaltung die UV-Strahlungsabgabe, sobald die Türe wieder geöffnet wird. Damit wird einerseits eine versehentliche Betätigung der UV-Lichtmittel bei geöffneter Türe vermieden und andererseits eine Fortführung der UV-Bestrahlung bei unbeabsichtigtem oder beabsichtigtem Öffnen der Türe verhindert. Diese Sicherheitsmaßnahmen machen ein fehlerhaftes Bedienen der erfindungsgemäßen Vorrichtung nahezu unmöglich.

Bevorzugt umfasst jede der Desinfektionsstationen zusätzlich Lichtschranken. Die Lichtschranken geben jeweils ein Signal an die Steuerungseinheit ab, wenn eine Desinfektionsstation mit Einkaufs-, Schub-, Transport- oder Gepäckwagen voll ist, bzw. wenn alle Wagen entnommen sind.

Außerdem kann jede Desinfektionsstation einen Bewegungsmelder umfassen, um eine Ansteuerung der Tür zu verhindern, solange sich Personen im Desinfektionsinnenraum aufhalten. Die Signale aller Sensoren werden in der übergeordneten Ablaufsteuerung ausgewertet. Die Ansteuerung der Türen und der Signalmittel erfolgt bevorzugt gleichzeitig, wenn die Entnahmestation leer ist, oder die Aufgabestation voll ist und sobald kein Signal des Bewegungsmelders im Innenraum einer der Stationen erfasst wird.

Weiters kann die Steuerungseinheit dermaßen ausgeführt sein, dass bei der Entkeimungsstation die Türe an der Vorderwand verriegelt ist. Sofern es sich bei der Tür um ein Rolltor handelt, kann dieses über einen elektrischen Antrieb und geregelt durch die Steuerungseinheit geschlossen bzw. geöffnet werden. Ein geschlossenes Rolltor lässt sich dann manuell nicht öffnen, wodurch ein versehentliches Öffnen der Türe dann nicht mehr möglich ist. Zusätzlich kann zur Unterbrechung des Desinfektionsvorgangs mithilfe der Steuerungseinheit die UV-Strahlung ausgeschalten werden, wodurch die Tür wieder entriegelt wird bzw. das Rolltor hochfährt und der Innenraum der Vorrichtung wieder zugänglich ist. Dadurch kann die Steuerung für die einzelnen Desinfektionsstationen durch die mit der Türsteuerung verbundene Sicherheitsschaltung getriggert werden.

Um eine versehentliche Bestrahlung zu vermeiden, können zusätzlich weitere Sicherheitsmaßnahmen zur erfindungsgemäßen Vorrichtung hinzugefügt werden. Beispielsweise kann zumindest ein Lichtmittel zur Abgabe von Licht im sichtbaren Spektralbereich, welches seriell mit den UV-Lichtmitteln verschaltet ist, eingebunden werden. Dieses zusätzliche Lichtmittel kann somit während der UV-Bestrahlung der Gegenstände in der Entkeimungsstation sichtbares Licht abstrahlen und ein visuelles Signal für die laufende Bestrahlung darstellen. Sollte die UV-Bestrahlung trotz geöffneter Türe weiterhin erfolgen, erkennt ein Nutzer aufgrund des sichtbaren Lichts sofort, dass die UV-Lichtmittel Licht abgeben und ein versehentliches Betreten des Innenraums kann verhindert werden. Für den größtmöglichen Schutz kann zum Beispiel an jedem verwendeten UV-Lichtmittel ein zusätzliches Lichtmittel zur Abgabe von sichtbarem Licht eingebaut und seriell verschaltet werden. Bei dem sichtbaren Licht kann es sich um blaues Licht handeln, welches sich stark vom Tageslicht unterscheidet und sofort erkannt wird. Das zusätzliche Lichtmittel kann in diesem Fall eine blaue LED sein. Darüber hinaus können an jeder Desinfektionsstation sowohl im Innenbereich als auch im Außenbereich des Rolltores Not-Aus Taster vorgesehen werden, um aus Sicherheitsgründen jederzeit eine Unterbrechung der UV-Bestrahlung zu ermöglichen.

Des Weiteren kann die Steuerungseinheit eine Anlaufstrombegrenzung sowie eine aktive Regelung zur Sicherstellung konstanter Bestrahlungsstärken umfassen. Der Anlaufstrombegrenzer kann die hohen Einschaltströme der LED-Treiber kompensieren und erlaubt eine vereinfachte elektrische Absicherung der Anlage.

Besonders bevorzugt erfolgt die UV-Bestrahlung zeitgesteuert, wobei die Zeitdauer einstellbar ist. Die Zeitdauer kann dabei wiederum über die Steuerungseinheit geregelt werden.

In einer bevorzugten Ausführungsvariante umfasst die Steuerungseinheit außerdem eine aktive Intensitätsregelung und einen UV-Sensor. Der UV-Sensor ist in den Steuerungskreis eingebunden. Damit lässt sich die Intensität der UV-Bestrahlung aktiv auf ein konstantes Niveau regeln. Somit sind unterschiedliche Desinfektionsprogramme realisierbar, und es ist sichergestellt, dass die gewünschte UV-Dosis erreicht wird.

In einer anderen Ausführungsvariante kann die Steuerungseinheit eine Intensitätssteuerung zur zeitabhängigen Leistungsanpassung aufweisen. Die Intensitätssteuerung erfolgt dann über eine Constant-Light-Output-Funktion der LED-Treiber, um den lebensdauerbedingten Strahlungsrückgang der LEDs auszugleichen und eine annähernd konstante UV-Dosis sicherzustellen. Die zeitabhängige Leistungsanpassung kompensiert damit den lebensdauerbedingten Lichtstromverlust der UV-C LEDs.

Eine Ausführungsvariante der erfindungsgemäßen Vorrichtung sieht eine reflektierende, vorzugsweise hochreflektierende, Auskleidung in den Desinfektionsinnenräumen vor. Die reflektierende Auskleidung minimiert wiederum die Schattenbildung. Außerdem ermöglicht sie das Verwenden einer geringeren Anzahl von UV-Lichtmitteln bzw. LED-Modulen ohne Einbußen der Desinfektionsleistung. Damit wird die Effizienz der Desinfektionsstation maximiert. Bevorzugt ist die Auskleidung an die UV-Strahlung angepasst. Bei Verwendung einer UV-C Strahlung kann eine spezielle Beschichtung für den UV-C Bereich verwendet werden, um die Bestrahlungseffizienz zu maximieren.

Außerdem kann auch der Boden im Desinfektionsinnenraum eine (hoch-)reflektierende Auskleidung aufweisen und somit eine Reflexion der UV-Strahlung auch vom Boden ermöglichen.

An der Außenseite Desinfektionsstationen kann eine Schutzverblechung angebracht werden. Die Schutzverblechung verhindert einerseits Strahlungsaustritte und schützt andererseits innenliegende Bauteile.

### DETAILLIERTE BESCHREIBUNG DER ERFINDUNG

Weitere Details und Vorteile der Erfindung ergeben sich aus den nachfolgenden Figuren und der dazugehörigen Figurenbeschreibung.
- Fig.1a-1b: zeigen eine Draufsicht auf die erfindungsgemäße Vorrichtung mit drei Desinfektionsstationen ohne die jeweiligen Deckenwände der Desinfektionsstationen.
- Fig. 2: zeigt die Desinfektionsstationen in einer perspektivischen Darstellung.
- Fig. 3: zeigt eine Vorderansicht einer Desinfektionsstation ohne Vorderwand (rechte Seite) und eine perspektivische Darstellung einer Desinfektionsstation mit geöffneter Türe (linke Seite).
- Fig. 4: zeigt eine perspektivische Darstellung der Desinfektionsstationen ohne Deckenwände.
- Fig. 5: zeigt ein LED-Modul der UV-Lichtquelle mit UV-C sowie einer blauen LED.
- Fig. 6: zeigt ein Trägerprofil samt montiertem LED-Modul in Schnittdarstellung.
- Fig. 7: zeigt eine Draufsicht auf die erfindungsgemäße Vorrichtung mit sechs Desinfektionsstationen ohne die jeweiligen Deckenwände der Desinfektionsstationen.

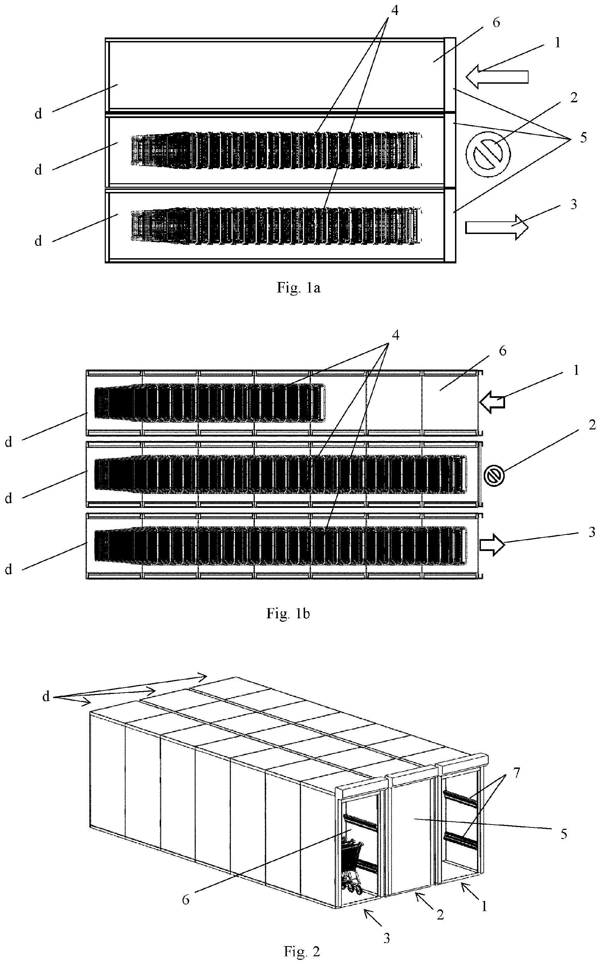

Die in Fig. 1a und 1b dargestellte Ausführungsform der erfindungsgemäßen Vorrichtung zur Desinfektion von Gegenständen wie insbesondere Einkaufswagen, Schubwagen, Transportwagen und Gepäckwagen mittels UV-Licht umfasst drei Desinfektionsstationen d, welche nebeneinander angeordnet sind. Jede der Desinfektionsstationen d umfasst einen Desinfektionsinnenraum 6, in welchen Einkaufswagen 4 hineingeschoben werden können. An der Vorderseite der Desinfektionsstationen d befindet sich eine verschließbare Türe 5, welche bevorzugt als Rolltor ausgeführt ist. In den Fig. 1a und 1b ist das Rolltor der oberen und unteren Desinfektionsstation d geöffnet, während die Türe der mittleren Desinfektionsstation d geschlossen ist.

In Fig. 1a und 1b entspricht die oberste Desinfektionsstation d der Aufnahmestation 1, die mittlere Desinfektionsstation d der Entkeimungsstation 2 und die unterste Desinfektionsstation d in Fig. 1 der Entnahmestation 3. In Fig. 1a sind dabei die Entkeimungsstation 2 und die Entnahmestation 3 bis zur maximalen Ladekapazität mit Einkaufswagen 4 befüllt, während sich in der Aufnahmestation 1 keine Einkaufswagen 4 befinden. Aus diesem Beladungszustand der Desinfektionsstationen d in Fig. 1a kann geschlossen werden, dass die mittlere Desinfektionsstation d zuvor noch der Aufnahmestation 1, die obere Desinfektionsstation d der Entnahmestation 3 und die untere Desinfektionsstation d der Entkeimungsstation 2 entsprach. Nachdem der letzte Einkaufswagen 4 aus der Entnahmestation 3 entnommen wurde bzw. die maximale Ladekapazität der Aufnahmestation 1 erreicht wurde, änderten sich die Funktionen der drei Desinfektionsstation d gemäß Fig. 1a.

Im Gegensatz dazu sind in Fig. 1b die Entnahmestation 3 und die Entkeimungsstation 2 bis zur maximalen Ladekapazität gefüllt, während die Aufnahmestation 1 zum Teil befüllt ist. Das heißt es wurden in Fig. 1b noch keine entkeimten Einkaufswagen 4 aus der Entnahmestation 3 entnommen, jedoch wurden bereits benutzte Einkaufwagen 4 in die Aufnahmestation 1 zurückgebracht.

Fig. 2 zeigt eine mögliche Ausführungsvariante der erfindungsgemäßen Vorrichtung mit drei Desinfektionsstationen d. Zur Desinfektion der Einkaufswagen 4 oder anderer Gegenstände umfasst jede der Desinfektionsstationen d mehrere UV-Lichtmittel 7. Wie in Fig. 2 ersichtlich befindet sich an der Vorderseite jeder der Desinfektionsstationen d eine Türe 5. Bei der Türe 5 kann es sich auch um ein Rolltor handeln. Bei geöffneter Türe 5 können Gegenstände in die Desinfektionsstation d eingebracht werden. In der in Fig. 2 gezeigten Ausführungsvariante der Vorrichtung ist die Türe 5 der mittleren Desinfektionsstation d, bei welcher es sich um die Entkeimungsstation 5 handelt, geschlossen, während die Türen 5 der linken und rechten Desinfektionsstationen d, bei welchen es sich um Entnahmestation 3 und Aufnahmestation 1 handelt, geöffnet sind. Die UV-Lichtmittel 7 sind dabei wie in Fig. 2 gezeigt bevorzugt an den Seitenwänden der Desinfektionsstationen d angebracht.

Die Wände der Desinfektionsstationen d können aus modularen Einzelsegmenten bestehen, welche anwendungsspezifisch skalierbar sind. Das heißt, die Größe einer Desinfektionsstation d ist beliebig anpassbar, indem die den Desinfektionsinnenraum 6 einschließenden Wände länger oder kürzer bzw. breiter oder schmäler ausgeführt werden. Daher beschränken sich die Gegenstände, welche mit der erfindungsgemäßen Desinfektionsstation d entkeimbar sind nicht nur auf Einkaufswagen 4, sondern auch auf andere Gegenstände, für welche eine Desinfektion mittels UV-Licht möglich ist. Die Größe des Desinfektionsinnenraum 6 lässt sich dann je nach Anwendung an die Gegebenheiten anpassen.

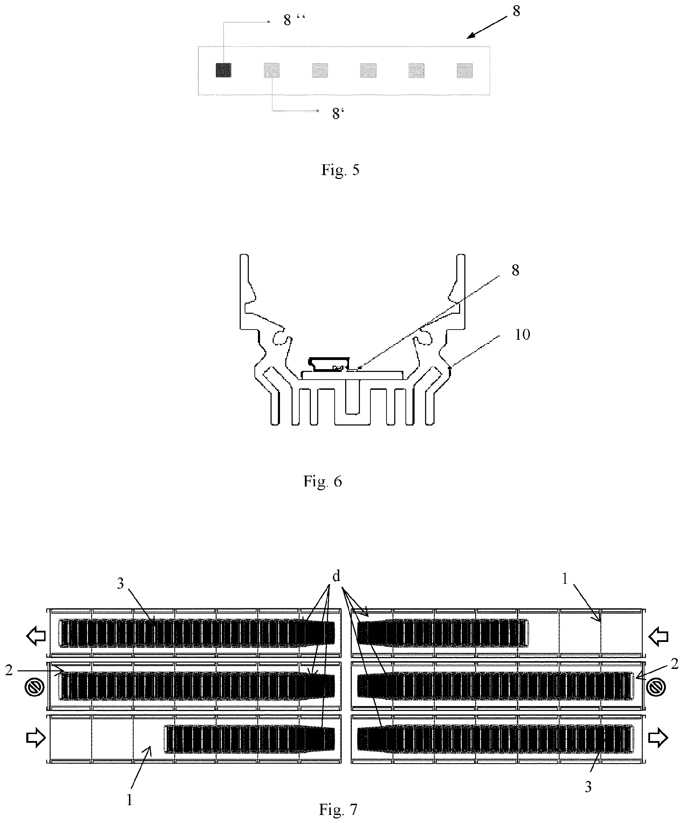

Bevorzugt umfassen die UV-Lichtmittel 7 mehrere LED-Module 8. Die LED-Module 8 können beispielsweise wie in Fig. 5 dargestellt ausgeführt sein und zumindest eine LED 8", welche Licht im sichtbaren Bereich abstrahlt und mehrere UV-LEDs 8' umfassen. Das LED-Modul 8 in Fig. 5 umfasst fünf UV-LEDs 8' und eine LED 8", welche bevorzugt blaues Licht abstrahlt.

Die LED-Module 8 können sehr einfach an Profilträgern 10, wie in Fig. 6 gezeigt, angebracht werden. Die genaue Ausgestaltung der Profilträger 10 ist dabei nicht auf die in Fig. 6 gezeigte Ausführungsvariante beschränkt und kann anwendungsspezifisch angepasst werden. Beispielsweise handelt es sich bei den Profilträgern 10 um Aluminium-Kühlprofile. Die LED-Module 8 können auf Aluminiumkernleiterplatten basieren um eine optimale thermische Behandlung der UV-C LEDs 8' zu garantieren. Die einzelnen LED-Module können seriell verschaltet werden und auf dem Profilträger 10 montiert werden. Die Profilträger 10 können dann auf einfache Weise an den Wänden der Desinfektionsstation d im Desinfektionsinnenraum 6 montiert werden.

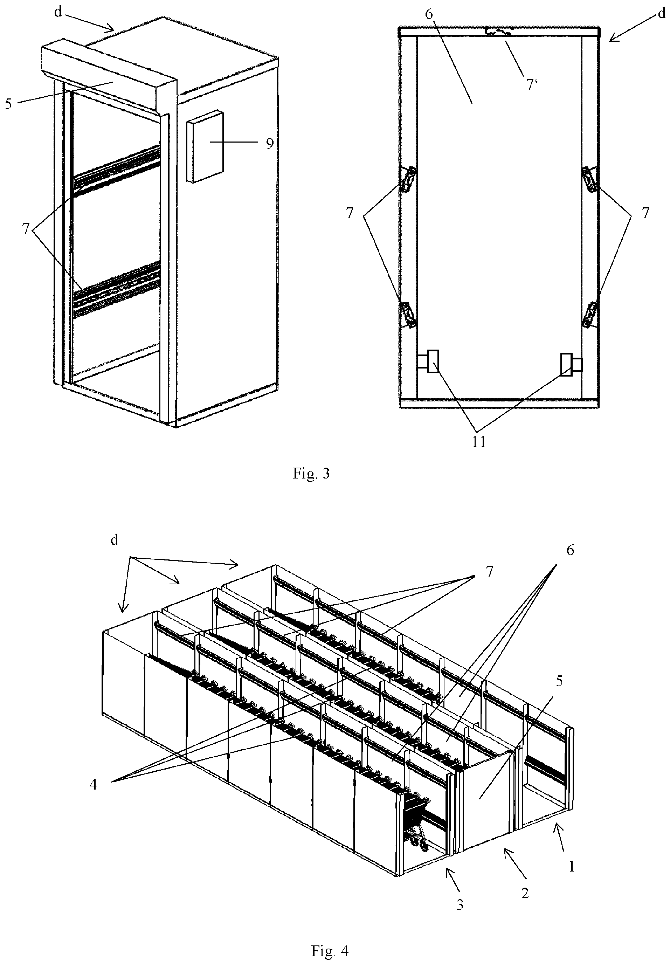

Wie in der Vorderansicht in Fig. 3 ersichtlich können auch mehrere UV-Lichtmittel 7 bzw. Profilträger 10 mit LED-Modulen 8 untereinander an den Wänden einer Desinfektionsstation d montiert werden. Zusätzlich können, wie in der Vorderansicht der Desinfektionsstation d auf der rechten Seite der Fig. 3 dargestellt, auch an der Decke Lichtmittel 7' angebracht werden. Dabei kann es sich ebenfalls um UV-Lichtmittel 7 handeln, um eine Bestrahlung der Gegenstände in der Desinfektionsstation d von oben zu ermöglich. Bei dem Lichtmittel 7' an der Decke kann es sich aber auch um eine Allgemeinbeleuchtung handeln. Dann wird vom Lichtmittel 7' sichtbares Licht ausgestrahlt, um eine Ausleuchtung im Inneren der Desinfektionsstation d zu erreichen. Eine Allgemeinbeleuchtung kann auch an einer der Seitenwände angebracht werden bzw. einfach in eines oder mehrere der UV-Lichtmittel 7 integriert werden.

Die Anordnung der UV-Lichtmittel 7 kann wie in Fig. 4 gezeigt für alle drei Desinfektionsstationen d an den Seitenwänden analog ausgeführt werden. Bei der Desinfektion von Einkaufswagen 4 mit der erfindungsgemäßen Vorrichtung befinden sich die wesentlichen zu entkeimenden Bereiche ca. auf mittlerer Höhe der Desinfektionsstation d. Daher sind in diesem Fall die UV-Lichtmittel 7 an den seitlichen Wänden der Desinfektionsstation d mittig angebracht bzw. derart geneigt, um eine direkte Bestrahlung der Einkaufswagen 4 zu erreichen. Eine geneigte Anordnung der UV-Lichtmittel 7 an den Seitenwänden ermöglicht eine Bestrahlung der Gegenstände in einer Desinfektionsstation d aus allen Richtungen.

Zusätzlich befindet sich, wie in Fig. 3 auf der linken Seite dargestellt, an der Desinfektionsstation d noch eine Steuerungseinheit 9, mit welcher die Lichtabgabe der UV-Lichtmittel 7 steuerbar ist. Bevorzugt befindet sich bei einer Vorrichtung mit drei Desinfektionsstationen d wie in den Fig. 1-2 und 4 an jeder der Desinfektionsstationen d eine Steuerungseinheit 9. Dabei kann nur eine Steuerungseinheit 9 die "Master-Steuerung" umfassen, welche für die übergeordnete Ablaufsteuerung zuständig ist.

Jede Steuerungseinheit 9 weist bevorzugt eine Sicherheitsschaltung auf, welche sicherstellt, dass die UV-Strahlung von den UV-Lichtmitteln 7 nur bei geschlossener Tür 5 emittiert wird. Die Steuerungseinheit 9 erlaubt auch eine aktive Regelung zur Sicherstellung konstanter Bestrahlungsstärken durch Integration eines UV-Sensors und steuert bevorzugt auch das Öffnen und Schließen der Türen 5. Außerdem können die zumindest drei Desinfektionsstationen d der erfindungsgemäßen Vorrichtung jeweils ein visuelles Signalmittel umfassen, dessen visuelles Signal ebenfalls von der Steuerungseinheit 9 gesteuert wird.

In Fig. 7 ist eine weitere Ausführungsvariante der erfindungsgemäßen Vorrichtung mit sechs Desinfektionsstationen d dargestellt. Dabei sind jeweils zwei Vorrichtungen mit drei Desinfektionsstationen d, welche jeweils in eine Aufnahme-, Entnahme- und Entkeimungsstation 1, 3, 2 aufgeteilt sind, nebeneinander angeordnet. Benutzte Wagen können somit in zwei verschiedenen Aufnahmestationen 1 abgestellt werden und entkeimte Wagen aus zwei verschiedenen Entnahmestationen 3 entnommen werden. Je nach Anzahl der Einkaufs-, Schub-, Transport- oder Gepäckwagen lässt sich dieses System auf beliebig viele Desinfektionsstationen d erweitern. Eine solche Anordnung kann besonders bevorzugt auch beim Einsatz verschiedener Wagentypen verwendet werden. In der in Fig. 7 gezeigten Anordnung der Desinfektionsstationen d können beispielsweise zwei verschiedene Wagentypen abgestellt, entkeimt und wieder entnommen werden. Dafür können Wagen eines ersten Typs in die erfindungsgemäße Vorrichtung mit drei Desinfektionsstationen d auf der linken Seite in Fig. 7 und Wagen eines zweiten Typs in der erfindungsgemäßen Vorrichtung mit drei Desinfektionsstationen d auf der rechten Seite in Fig. 7 abgestellt und entnommen werden.

Ein wesentlicher Vorteil der erfindungsgemäßen Vorrichtung mit zumindest drei Desinfektionsstationen d, wobei jede der Desinfektionsstationen d entweder die Funktion der Aufnahme-, Entnahme- oder Entkeimungsstation 1, 2, 3 übernimmt, ist das Integrieren der Desinfektion von benutzten Wagen 4 in das den Kunden bekannte und etablierte Konzept der Entnahme und Abgabe von Wagen 4 oder dergleichen in Supermärkten oder anderen Geschäften und Infrastrukturen. Die Entkeimung erfordert daher einerseits keinen Mehraufwand bei den Kunden und andererseits keine Anpassung der Kunden an neue Gegebenheiten, welche schnell zu einer fehlerhaften Ausführung der Desinfektion führen könnten. Außerdem ist auch von Seiten der Geschäfte bzw. Infrastrukturen kein zusätzlicher Personalaufwand notwendig, da die Steuerungseinheit 9 das komplette Verfahren regelt. Die erfindungsgemäße Vorrichtung bietet Supermärkten, anderen Geschäften und Infrastrukturen, in welchen Einkaufs-, Schub-, Transport- oder Gepäckwagen 4 verwendet werden, eine äußerst effiziente und kostensparende Möglichkeit eine Desinfektion der Wagen 4 nach jeder Benutzung zu realisieren.

## Patentansprüche

1. Verfahren zur Desinfektion von Gegenständen, insbesondere Wagen (4) wie Einkaufswagen, Schubwagen, Transportwagen oder Gepäckwagen mittels UV-Licht,
wobei zumindest drei Desinfektionsstationen (d) vorgesehen sind,
wobei jede der Desinfektionsstationen (d) - vorzugsweise mehrere - UV-Lichtmittel (7), einen Desinfektionsinnenraum (6) und eine verschließbare Türe (5), vorzugsweise ein Rolltor an der Vorderseite umfasst,
wobei die UV-Lichtmittel (7) an den Innenwänden der Desinfektionsstation (d) angebracht sind, **dadurch gekennzeichnet, dass**
die zumindest drei Desinfektionsstationen (d) jeweils in eine Aufnahmestation (1) zur Aufnahme benutzter Gegenstände, vorzugsweise Wagen (4), eine Entnahmestation (3) zur Entnahme entkeimter Gegenstände, vorzugsweise Wagen (4) und eine Entkeimungsstation (2) zur Desinfektion der Gegenstände, vorzugsweise Wagen (4) aufgeteilt werden,
wobei während die Tür (5) der Entkeimungsstation (2) geschlossen ist, die Türen (5) der Entnahmestation (3) und der Aufnahmestation (1) geöffnet sind,
wobei bei geöffneter Türe (5) die Lichtabgabe durch die UV-Lichtmittel (7) deaktiviert und bei geschlossener Türe (5) die Lichtabgabe durch die UV-Lichtmittel (7) zumindest zeitweise aktiviert wird, wobei weiters die Schritte vorgesehen sind:
• Einschieben von benutzten Gegenständen, vorzugsweise Wagen (4) in die Aufnahmestation (1), wobei die Gegenstände, vorzugsweise Wagen (4) vorzugsweise ineinandergeschoben werden und Entnahme von entkeimten Gegenständen, vorzugsweise Wagen (4) aus der Entnahmestation (3), während in der Entkeimungsstation (2) benutzte Gegenstände, vorzugsweise Wagen (4) mit von den UV-Lichtmitteln (7) abgestrahlten UV-Licht bestrahlt werden, solange bis entweder die maximale Ladekapazität der Aufnahmestation (1) erreicht ist oder die Entnahmestation (3) keine Gegenstände, vorzugsweise Wagen (4) mehr beinhaltet,
• Schließen der Türe (5) und Aktivierung der UV-Lichtmittel (7) der Aufnahmestation (1) und Öffnen der Türe (5) der Entkeimungsstation (2),
• Starten bei Schritt eins, wobei die vormalige Entnahmestation (3) der Aufnahmestation (1), die vormalige Aufnahmestation (1) der Entkeimungsstation (2) und die vormalige Entkeimungsstation (2) der Entnahmestation (3) entspricht.

2. Verfahren nach Anspruch 1, **gekennzeichnet durch** ein vorzugsweise visuelles Signalmittel an der Vorderseite jeder der zumindest drei Desinfektionsstationen (d), wobei das Signalmittel zumindest zwei verschiedene Signale abgibt, wobei eine Steuerungseinheit (9) zeitgleich mit dem Schließen der Türe (5) der Aufnahmestation (1) und dem Öffnen der Türe (5) der Entkeimungsstation (2) die von den Signalmitteln abgegebenen Signale ändert.

3. Verfahren nach einem der Ansprüche 1 oder 2, wobei die UV-Lichtmittel (7) in der Entkeimungsstation (2) entweder beim Öffnen der Türe (5) oder nach einer eingestellten Zeit deaktiviert werden.

4. Vorrichtung zur Desinfektion von insbesondere Einkaufswagen, Schubwagen, Transportwagen oder Gepäckwagen mittels UV-Licht umfassend zumindest drei Desinfektionsstationen (d), wobei jede der Desinfektionsstationen (d)
• einen Desinfektionsinnenraum (6),
• eine verschließbare Türe (5), vorzugsweise ein Rolltor an der Vorderseite,
• mehrere UV-Lichtmittel (7), welche an den Innenwänden angebracht sind,
• vorzugsweise eine Allgemeinbeleuchtung,
• und eine Steuerungseinheit (9)
umfasst,
wobei die Steuerungseinheit (9) im Betriebszustand
(i) die drei Desinfektionsstationen (d) in Aufnahmestation (1), Entnahmestation (3) und Entkeimungsstation (2) unterteilt;
(ii) die Tür (5) der Entkeimungsstation (2) geschlossen hält und die Türen (5) der Aufnahmestation (1) und der Entnahmestation (3) geöffnet hält;
(iii) die Lichtabgabe der UV-Lichtmittel (7) steuert, wobei bei geöffneter Türe (5) die Lichtabgabe durch die UV-Lichtmittel (7) deaktiviert ist und wobei bei geschlossener Türe (5) die Lichtabgabe durch die UV-Lichtmittel (7) zumindest zeitweise aktiviert ist.

5. Vorrichtung nach Anspruch 4, wobei die Steuerungseinheit (9) das Schließen und Öffnen der Türen (5) steuert, wobei die Türe (5) der Aufnahmestation (1) schließt sobald entweder die maximale Ladekapazität der Desinfektionsstation (d) erreicht ist oder die Entnahmestation (3) leer ist, wobei die Tür (5) der Entkeimungsstation (2) öffnet sobald die Tür (5) der Aufnahmestation (1) schließt, wobei die Entkeimungsstation (2) dann zur Entnahmestation (3), die Aufnahmestation (1) zur Entkeimungsstation (2) und die Entnahmestation (3) zur Aufnahmestation (1) wird.

6. Vorrichtung nach einem der Ansprüche 4 bis 5, **gekennzeichnet durch** ein Signalmittel, mit welchem verschiedene Signale abgebbar sind, wobei das Signalmittel von der Steuerungseinheit (9) gesteuert wird, sodass die Desinfektionsstationen (d) als Aufnahmestation (1), Entnahmestation (3) und Entkeimungsstation (2) kennzeichenbar sind.

7. Vorrichtung nach Anspruch 6, **dadurch gekennzeichnet, dass** das Signalmittel ein visuelles Signalmittel ist, welches vorzugsweise eine Anzeigetafel umfasst.

8. Vorrichtung nach einem der Ansprüche 4 bis 7, **dadurch gekennzeichnet, dass** an jeder der Seitenwände des Desinfektionsinnenraums (6) zumindest ein UV-Lichtmittel (7) angebracht ist.

9. Vorrichtung nach einem der Ansprüche 4 bis 8, wobei es sich bei dem von den UV-Lichtmitteln (7) ausgesandten Licht um UV-C Licht mit einer Wellenlänge von vorzugsweise 250 bis 280 nm handelt.

10. Vorrichtung nach einem der Ansprüche 4 bis 9, **dadurch gekennzeichnet, dass** die UV-Lichtmittel (7) LED-Module (8) umfassen, wobei ein LED-Modul (8) zumindest eine UV-LED (8'), vorzugsweise mehrere UV-LEDs (8'), und vorzugsweise zumindest eine LED (8") zur Abgabe von Licht im sichtbaren Spektralbereich umfasst.

11. Vorrichtung nach einem der Ansprüche 4 bis 10, wobei die Steuerungseinheit (9) eine Sicherheitsschaltung umfasst, wobei die Sicherheitsschaltung die Lichtabgabe der UV-Lichtmittel (7) bei offener Türe (5) unterbricht.

12. Vorrichtung nach einem der Ansprüche 4 bis 11, wobei die Steuerungseinheit (9) eine aktive Intensitätsregelung samt UV-Sensor umfasst, wobei mit der aktiven Intensitätsregelung der Lichtstrom im UV-Spektrum aktiv regelbar ist.

13. Vorrichtung nach einem der Ansprüche 4 bis 11, wobei die Steuerungseinheit (9) eine Intensitätssteuerung zur zeitabhängigen Leistungsanpassung umfasst, wobei die Intensitätssteuerung den lebensdauerbedingten Lichtstromverlust der UV-Lichtmittel (7) kompensiert.

14. Vorrichtung nach einem der Ansprüche 4 bis 13, **gekennzeichnet durch** eine reflektierende, vorzugsweise hochreflektierende Auskleidung an den Innenwänden des Desinfektionsinnenraums (6) der zumindest drei Desinfektionsstationen (d).

15. Vorrichtung nach einem der Ansprüche 4 bis 14, **gekennzeichnet durch** eine Schutzverblechung an der Außenseite der zumindest drei Desinfektionsstationen (d).
